# FASCICULE DE BREVET EUROPEEN

(11) **EP 1 891 443 B1**
(45) Date de publication et mention de la délivrance du brevet: **20.01.2010**
(21) Numéro de dépôt: 06778939.6
(22) Date de dépôt: 14.06.2006
(51) Int. Cl.: G01N 33/574

(54) **PROCÉDÉ POUR LE CRIBLAGE DE SUBSTANCES ANTI-CANCÉREUSES , TROUSSE OU KIT POUR LA MISE EN OEUVRE DU PROCÉDÉ**
VERFAHREN ZUR UNTERSUCHUNG VON SUBSTANZEN GEGEN KREBS, SATZ ODER AUSRÜSTUNG ZUR UMSETZUNG DES VERFAHRENS
METHOD FOR SCREENING ANTICANCER SUBSTANCES, SET OR KIT FOR IMPLEMENTING SAID METHOD

(30) Priorité: 14.06.2005 FR 0551616
(43) Date de publication de la demande: 27.02.2008
(73) Titulaire: Centre National de la Recherche Scientifique, 75794 Paris Cedex 16 (FR)
(72) Inventeur: ROUX, Pierre, F-34980 Saint-Gely-du-Fesc (FR); DE TOLEDO, Marion, F-34080 Montpellier (FR)
(74) Mandataire: Catherine, Alain
(86) Numéro de dépôt international: PCT/FR2006/050561
(87) Numéro de publication internationale: WO 2006/134305

(56) Documents cités:
- WO-A-2004/062620
- US-A1- 2004 192 744
- US-A1- 2005 089 896
- PALMER HECTOR G ET AL: "Vitamin D3 promotes the differentiation of colon carcinoma cells by the induction of E-cadherin and the inhibition of beta-catenin signaling" JOURNAL OF CELL BIOLOGY, vol. 154, no. 2, 23 juillet 2001 (2001-07-23), pages 369-387, XP002378341 ISSN: 0021-9525
- CARTER ORIANNA ET AL: "The dietary phytochemical chlorophyllin alters e-cadherin and beta-catenin expression in human colon cancer cells" JOURNAL OF NUTRITION, vol. 134, no. 12, Suppl. S, décembre 2004 (2004-12), pages 3441S-3444S, XP002378342 ISSN: 0022-3166
- MUELLER NICOLE ET AL: "Smad4 induces the tumor suppressor E-cadherin and P-cadherin in colon carcinoma cells" ONCOGENE, vol. 21, no. 39, 5 septembre 2002 (2002-09-05), pages 6049-6058, XP002378343 ISSN: 0950-9232

### SEQUENCE LISTING

<110> Centre National de la Recherche Scientifique
<120> Procédé pour le criblage de substances anticancéreuses; trousse ou kit pour la mise en oeuvre du procédé
<130> T180FR
<160> 6
<170> Patent In version 3.1
<210> 1
   <211> 882
   <212> PRT
   <213> Homo sapiens
<400> 1
<210> 2
   <211> 781
   <212> PRT
   <213> Homo sapiens
<400> 2
<210> 3
   <211> 9
   <212> PRT
   <213> séquence artificielle
<400> 3
   Tyr Pro Tyr Asp Val Pro Asp Tyr Ala
   1 5
<210> 4
   <211> 8
   <212> PRT
   <213> Séquence artificielle
<400> 4
   Asp Tyr Lys Asp Asp Asp Asp Lys
   1 5
<210> 5
   <211> 23
   <212> PRT
   <213> Séquence artificielle
<400> 5
   Met Asp Tyr Lys Asp His Asp Gly Asp Tyr Lys Asp His Asp Ile Asp
   1 5 10 15
   Tyr Lys Asp Asp Asp Asp Lys
   20
<210> 6
   <211> 11
   <212> PRT
   <213> Séquence artificielle
<400> 6
   Met Glu Gln Lys Leu Ile Ser Glu Glu Asp Leu
   1               5                   10

## Description

### RESUME DE L'INVENTION

La présente invention se rapporte au domaine de l'identification de molécules d'intérêt thérapeutique pour le traitement des cancers.

La présente invention a pour objet un procédé pour le criblage de substances anti-cancéreuses anti-métastatiques capables d'inverser le phénotype métastatique vers un phénotype non métastatique comprenant les étapes suivantes :
a) obtenir une culture de cellules tumorales n'exprimant pas la E-cadhérine sur leur membrane cellulaire ;
b) mettre en contact les cellules obtenues à l'étape a) avec une substance ou une combinaison de substances candidate(s) ;
c) détecter, dans la culture de cellules obtenue à la fin de l'étape b), la présence de E-cadhérine à la surface cellulaire ;
d) sélectionner positivement la substance ou la combinaison de substances candidate(s) lorsque la E-cadhérine a été détectée à l'étape c).

L'invention a aussi pour objet une trousse ou kit pour le criblage de substances anti-cancéreuses anti-métastatiques capables d'inverser le phénotype métastatique vers un phénotype non métastatique comprenant :
a) des cellules métastatiques n'exprimant pas la E-cadhérine sur leur membrane cellulaire ;
b) un composé ligand de la E-cadhérine.

L'invention est aussi relative à l'utilisation d'un composé ligand de la partie extra-membranaire de la E-cadhérine, pour le criblage *in vitro* de substances anti-cancéreuses anti-métastatiques capables d'inverser le phénotype métastatique vers un phénotype non métastatique.

### ART ANTERIEUR

On sait que, dans certains cancers, les cellules tumorales ont non seulement une forte capacité à proliférer, mais aussi une forte capacité à détruire les tissus et à s'infiltrer dans les tissus voisins, y compris dans les vaisseaux sanguins et les vaisseaux lymphatiques, puis à migrer par la circulation sanguine vers des tissus localisés à des endroits du corps très éloignés de la tumeur primaire. Ainsi, dans certains cancers, les cellules tumorales ont la capacité de circuler puis de former des tumeurs secondaires, aussi appelées métastases, dans des tissus distants de la tumeur primaire.

Comme mentionné ci-dessus, la formation de métastases est un phénomène physiologique à étapes multiples, au cours duquel des cellules tumorales se détachent de la tumeur primaire, envahissent la matrice cellulaire, pénètrent au travers des vaisseaux sanguins, entrent dans le système vasculaire par intravasation, puis arrêtent leur migration dans la circulation sanguine ou lymphatique à un site distant, sortent de la circulation par extravasation, puis se fixent dans un tissu distant et prolifèrent pour former une tumeur secondaire.

Différentes molécules sont susceptibles de jouer un rôle dans la formation de métastases, y compris certains récepteurs de chimiokines ou encore des intégrines.

Notamment, on a montré dans l'état de la technique que certaines protéines de la famille des cadhérines étaient susceptibles d'être impliquées dans les mécanismes physiologiques conduisant à la formation de métastases.

La famille des cadhérines est une famille de protéines qui possèdent une activité de régulation de l'adhésion des cellules épithéliales, endothéliales, neurales ou cancéreuses. Différents types de cadhérines sont exprimés selon les tissus, telles que (i) la N-cadhérine, qui est exprimée majoritairement par les cellules neurales, les cellules endothéliales et divers types de cellules cancéreuses ; (ii) le E-cadhérine, qui est exprimée exclusivement par les cellules épithéliales ; (iii) la P-cadhérine, qui est trouvée au niveau de la peau, chez l'homme; (iv) la R-cadhérine, qui est exprimée dans la rétine.

Du fait de l'implication des cadhérines dans l'adhésion cellulaire, on a proposé dans l'état de la technique divers procédés et produits capables de moduler l'activité de régulation de l'adhésion cellulaire de ces protéines. Ainsi, dans la demande de brevet américain n° US 2003/0109454, on a proposé de modifier les fonctions des cadhérines à l'aide d'agents modulateurs protéiques comprenant au moins un motif « HAV », motif qui est essentiel à la liaison entre deux molécules de cadhérines, encore appelée liaison homotypique. Selon cette demande de brevet américain, de tels agents modulateurs seraient capables d'agir sur l'adhésion cellulaire et ainsi faciliter le passage de médicaments au travers des tissus. La demande de brevet américain n° US 2003/109454 décrit aussi un procédé permettant de sélectionner des agents modulant l'adhésion cellulaire, en sélectionnant positivement les composés candidats se fixant sur des anticorps dirigés contre une séquence contenant un motif d'adhésion « HAV ». Egalement, la demande de brevet américain n° US 2002/0192724 décrit l'utilisation de protéines, en particulier d'anticorps, qui se fixent sur certaines régions extracellulaires des cadhérines, en vue de moduler l'adhésion des lymphocytes T aux cellules exprimant des cadhérines.

On peut aussi citer le brevet américain n° US 6,468,790 qui décrit des séquences de protéines constituant des marqueurs métastatiques pour les cancers du sein et du colon. Parmi les nombreux marqueurs décrits, on cite la E-cadhérine, du fait que (i) l'ARN messager codant la E-cadhérine est retrouvé dans les extraits cellulaires d'une lignée de cancer du sein non métastatique (MCF7), mais que (ii) l'ARN messager codant la E-cadhérine n'est pas retrouvé dans les extraits cellulaires de deux lignées de cancer du sein non métastatiques (MDA-MB-231 et MDA-MB-435). Dans le brevet américain n° US 6,468,790, on propose de détecter le niveau d'expression de ces marqueurs protéiques, ou le niveau d'expression des gènes ou ARN messagers correspondants, afin de déterminer les cancers susceptibles de s'étendre par formation de métastases. Le brevet américain n° US 6,468,790 propose aussi des procédés de criblage de médicaments susceptibles de posséder un effet anti-métastatique. Selon ces procédés de criblage, on incube le composé candidat à tester avec des cellules cancéreuses et on détermine le niveau d'expression du marqueur protéique, ou du gène ou de l'ARN messager correspondant. Le niveau d'expression du marqueur protéique est réalisé soit (i) par incorporation cellulaire d'acides aminés marqués, puis détection des protéines marqueur marquées sur un gel de polyacrylamide, soit (ii) en détectant les protéines marqueurs avec des anticorps spécifiques, dans des essais d'immuno-empreintes (aussi appelés « Western blots »). Le niveau d'expression du gène codant le marqueur protéique d'intérêt est réalisé par l'analyse des ARN messagers, selon la technique connue appelée « Northem blot ».

Cependant, les mécanismes de l'initiation et du développement des métastases cancéreuses ne peut être expliqué du fait de l'implication de la seule E-cadhérine, et l'on sait que de nombreux autres facteurs sont impliqués dans ces mécanismes. Ainsi, bien que, dans le brevet américain n° US 6,468,790, on montre une inactivation de l'expression de protéines comme la E-cadhérine par des cellules de cancer métastatique du sein, il n'est présenté aucun résultat susceptible de montrer (i) qu'une inactivation de l'expression de la E-cadhérine est susceptible de transformer une cellule cancéreuse en cellule cancéreuse métastatique, ou à l'inverse (ii) qu'une activation de l'expression de la E-cadhérine est susceptible de transformer une cellule cancéreuse métastatique en cellule non-métastatique.

Palmer, H. et al.: Journal of Cell Biology, vol. 154, no. 2. p. 369-387 décrit les effets d'un dérivé de la vitamine D3 (1α,25(OH)₂D₃ sur la differenciation de cellules dérivées d'un carcinome du colon. Mais le composé 1α,25(OH)₂D₃ n'induit pas de modification phénotypique chez la lignée SW620 (choisi comme lignée cellulaire témoin).

Ainsi, à la connaissance du demandeur, il n'existe pas à ce jour un quelconque moyen technique qui permettrait de déterminer le caractère potentiellement métastatique d'une cellule cancéreuse.

De plus, à la connaissance du demandeur, il n'existe pas de procédé permettant de cribler, dans des conditions proches de la physiologie cellulaire, des composés anti-métastatiques.

Or, la formation de métastases de cellules cancéreuses est un mauvais pronostic pour le patient, car cette situation nécessite, du fait de la dispersion géographique des métastases, des traitements anti-métastatiques exclusivement par voie systémique.

II existe donc un besoin dans l'état de la technique d'identifier de nouveaux composés capables d'inhiber la migration des cellules tumorales et de prévenir ainsi la formation de métastases, et en conséquence de procédés de criblage fiables permettant de sélectionner ces nouveaux composés anti-métastatiques.

### DESCRIPTION DE L'INVENTION

La présente invention fournit un procédé pour le criblage de substances anti-métastatiques.

De manière surprenante, le demandeur a montré qu'il existe une corrélation stricte entre l'induction de la perte du pouvoir migratoire et du pouvoir d'invasion de cellules initialement métastatiques, et l'induction de la formation de jonctions intercellulaires impliquant la présence de E-cadhérine. La démonstration par le demandeur de cette corrélation surprenante lui a permis de concevoir et mettre au point un procédé pour le criblage de substances anti-métastatiques qui met en oeuvre des cellules cancéreuses initialement métastatiques, et qui comprend une étape au cours de laquelle on détecte la présence de E-cadhérine exposée à la surface des membranes des cellules préalablement traitées par une substance ou une combinaison de substances candidates.

La présente invention a pour objet un procédé pour le criblage de substances anti-cancéreuses anti-métastatiques capables d'inverser le phénotype métastatique vers un phénotype non-métastatique comprenant les étapes suivantes :
a) obtenir une culture de cellules métastatiques n'exprimant pas la E-cadhérine sur leur membrane cellulaire ;
b) mettre en contact les cellules obtenues à l'étape a) avec une substance ou une combinaison de substances candidate(s) ;
c) détecter, dans la culture de cellules obtenue à la fin de l'étape b), la présence de E-cadhérine à la surface cellulaire ;
d) sélectionner positivement la substance ou la combinaison de substances candidate(s) lorsque la E-cadhérine a été détectée à l'étape c).

Par « substances anti-cancéreuses », on entend selon l'invention des substances capables de faire réverser des cellules cancéreuses ayant un phénotype de cellules métastatiques, vers un phénotype non-métastatique. Parmi les substances anti-cancéreuses susceptibles d'être sélectionnées positivement avec le procédé de l'invention, certaines d'entre elles ne vont pas modifier la capacité de prolifération des cellules cancéreuses initialement métastatiques. Mais d'autres substances anti-cancéreuses susceptibles d'être sélectionnées positivement avec le procédé de l'invention pourront, à la fois, induire une réversion du phénotype métastatique vers un phénotype non-métastatique et inhiber ou bloquer la capacité de prolifération des cellules initialement cancéreuses et métastatiques.

Du fait de la corrélation stricte qui a été montrée selon l'invention entre le blocage du pouvoir invasif des cellules cancéreuses et la présence de E-cadhérine exposée à la surface des membranes cellulaires, le procédé ci-dessus permet le criblage de substances candidates anti-métastatiques, dans des conditions dans lesquelles les cellules testées sont dans une situation proche de leur situation physiologique *in vivo.*

En particulier, le procédé de l'invention rend possible le criblage de substances anti-métastatiques, qui n'agissent pas directement sur l'expression de la E-cadhérine. Notamment, le procédé de criblage selon l'invention permet de sélectionner positivement des substances anti-métastatiques, qui sont actives sur des cibles variées dans la cellule, dès lors que leur capacité anti-métastatique est dans tous les cas vérifiée par leur capacité à faire réverser le phénotype des cellules traitées vers un phénotype de cellule non-métastatique, par formation des jonctions intercellulaires, ce qui est déterminé par la détection de la présence de E-cadhérine à la surface des membranes cellulaires.

Le procédé ci-dessus permet de cribler les substances candidates directement sur les cellules vivantes, et permet ainsi de sélectionner positivement les substances ayant un effet physiologique d'inhibition ou de blocage de la capacité des cellules à former des métastases.

Comme cela est montré dans les exemples, le procédé de l'invention est particulièrement adapté pour le criblage de substances anti-métastatiques actives sur les cellules cancéreuses métastatiques dérivées de cellules épithéliales. On a ainsi montré que le procédé de l'invention permet de sélectionner avec succès des substances faisant réverser des cellules cancéreuses mésenchymateuses vers un phénotype de cellule épithéliale capables à nouveau de former des jonctions intercellulaires.

Un autre avantage du procédé selon l'invention est que ce procédé permet de ne sélectionner positivement qu'exclusivement les substances anti-métastatiques, qui ne sont pas toxiques pour les cellules. En effet, toutes les substances candidates qui sont toxiques pour les cellules, du fait de la mort cellulaire, ne pourront pas induire de modification physiologique des cellules, et ne pourront notamment pas induire de réversion vers le phénotype non-métastatique, avec re-formation de jonctions intercellulaire en présence de E-cadhérine. Les substances candidates cytotoxiques ne seront donc jamais sélectionnées positivement avec le procédé de l'invention.

A l'étape a) du procédé, on utilise avantageusement des cellules tumorales métastatiques n'exprimant pas la E-cadhérine, qui dérivent de cellules épithéliales. En général, ces cellules métastatiques consistent en des cellules d'origine épithéliale qui ont perdu la morphologie cellulaire de type épithélial, et qui ont acquis des propriétés de motilité cellulaire du fait de remaniements du cytosquelette et du fait de la formation de nouveaux types de contact avec la matrice extracellulaire. L'ensemble de ces transformations phénotypiques de la cellule épithéliale initiale constitue la transition épithélio-mésenchymateuse (EMT).

Ainsi, à l'étape a) du procédé, on utilise avantageusement des cellules cancéreuses métastatiques, de préférence d'origine épithéliale, qui peuvent être choisies parmi les cellules établies en lignées cellulaires, ou qui peuvent être choisies parmi les cellules cancéreuses métastatiques en culture primaire, qui proviennent d'un prélèvement cellulaire ou d'une biopsie à partir d'un patient atteint d'un cancer.

Parmi les lignées cellulaires cancéreuses métastatiques, on peut utiliser des cellules tumorales ayant la capacité de former des métastases qui sont choisies parmi les lignées cellulaires suivantes : SW620 (ATCC n° CCL-227), SKCo-1 (ATCC n° HTB-39), et CoLo205 (ATCCn° CCL-222).

A l'étape a) du procédé, le nombre de cellules ensemencées par conteneur de culture varie selon le conteneur utilisé et, le cas échéant, le type de cellules utilisées, en particulier selon la capacité de prolifération des cellules cancéreuses utilisées. A titre indicatif, lorsque les cellules cancéreuses sont ensemencées dans des plaques de culture conventionnelles de 96 puits, on peut ensemencer les cellules à raison de 2000 à 30 000 cellules par puits de culture, mieux de 5000 à 20 000 cellules par puits de culture. L'homme du métier de référera à ses connaissances générales pour adapter le nombre de cellules, en fonction du type de conteneur de culture et du type de cellules cancéreuses utilisées.

Ainsi, le procédé de l'invention est particulièrement adapté pour le criblage de substances candidates ayant la capacité de bloquer la capacité à former des métastases de cellules cancéreuses dérivées de cellules épithéliales. Grâce au procédé de l'invention, on peut cribler des substances physiologiquement actives anti-métastatiques susceptibles d'être utilisées dans la fabrication de médicaments utiles dans la prévention ou le traitement de cancers connus pour se disséminer sous la forme de métastases, en particulier des cancers des cellules épithéliales tels les cancers du sein, les cancer colo-rectaux, les cancers ovariens, les cancers de la vessie, les cancers du col de l'utérus, les cancers de la peau y compris les cancers basocellulaires, les cancers de l'estomac, les tumeurs épithéliales du thymus, les cancers de la prostate, des testicules, du poumon, de la gorge, du pancréas, du foie, de la vésicule biliaire, et les cancers de la vessie.

A l'étape b) du procédé, on incube les cellules avec une substance ou une combinaison de substances candidate à tester. Dans une combinaison de substances candidates à tester, une ou plusieurs de ces substances peuvent déjà être connues pour leur pouvoir anti-métastatique. Par exemple, on peut utiliser, dans une combinaison de substances candidates à tester, au moins une substance dont la capacité anti-métastatique est connue, afin de déterminer l'existence d'une éventuelle synergie d'action entre la substance d'activité connue et la ou les autres substances de ladite combinaison de substances.

A l'étape b), la culture de cellules est mise en contact avec la ou les substance(s) à tester pendant une durée variable, qui peut varier, à titre illustratif, de 30 minutes à 72 heures.

En général, l'étape b) est réalisée en utilisant une série de cultures de cellules, par exemple dans des puits de micro-plaques de culture cellulaire d'un type connu, et en incubant chaque puits ou chaque sous-série de puits avec des concentrations variables de la ou des substance(s) à tester. Avantageusement, un puits ou une sous-série de puits n'est incubé qu'avec le milieu de culture et sert de témoin négatif. Avantageusement, un puits ou une sous-série de puits est incubé avec une concentration ou une série de concentrations connues d'une substance connue pour son pouvoir anti-métastatique et sert de témoin positif.

A l'étape c) du procédé, la détection de la présence de la E-cadhérine à la surface des membranes des cellules en culture peut être réalisée par l'homme du métier à l'aide de toute technique de détection connue.

Selon un certain mode de réalisation, l'étape c) de détection est réalisée sur les cellules en culture, sans traitement préalable des cultures cellulaires.

Selon un autre mode de réalisation, l'étape c) de détection est réalisée après un traitement préalable des cellules en culture, de préférence un traitement de fixation des cellules en culture, à l'aide de tout agent ou de toute combinaison d'agents de fixation cellulaire connus de l'homme du métier. A titre illustratif, au début de l'étape c) du procédé, les cellules en culture sont fixées avec de la formaline, préalablement à l'étape de détection de la E-cadhérine proprement dite. Les cellules sont avantageusement incubées avec l'agent de fixation pendant une durée appropriée, selon l'agent utilisé, par exemple pendant une durée variant de 1 minute à 1 heure. Pour la formaline, une durée d'incubation avec l'agent de fixation variant de 5 minutes à 30 minutes, mieux de 5 minutes à 15 minutes est adaptée.

Avantageusement, la détection de la présence de la E-cadhérine extra-membranaire est réalisée, à l'étape c) du procédé, en mettant en contact les cellules obtenues à la fin de l'étape b) avec un composé ligand de la E-cadhérine.

Lorsqu'on utilise un agent de fixation des cellules, on peut, dans un mode de réalisation particulier, traiter les cellules fixées avec des protéines de saturation en suspension dans un tampon, telle que de la sérum albumine, y compris de la sérum albumine bovine (BSA), pendant une durée de 2 à 30 minutes, par exemple de 10 minutes, afin d'éviter, ou tout au moins de réduire, les événements de fixation non spécifique du composé ligand de la E-cadhérine.

Par composé « ligand » de la E-cadhérine, on entend selon l'invention un composé qui a la capacité à se fixer spécifiquement sur la E-cadhérine exprimée sur les membranes cellulaires. En général, ledit composé « ligand » se fixe sur le domaine extra-membranaire de la E-cadhérine.

Dans la E-cadhérine de séquence SEQ ID N° 1, la partie peptidique extra-membranaire débute à l'acide aminé en position 155 et se termine à l'acide aminé en position 697 de la séquence SEQ ID N° 1.

En général, un composé ligand qui se fixe sur la E-cadhérine est choisi parmi les acides nucléiques ou les polypeptides qui reconnaissent la E-cadhérine.

A titre illustratif, un acide nucléique ligand de la E-cadhérine peut être aisément sélectionné par l'homme du métier en mettant en oeuvre la technique SELEX. La méthode SELEX consiste en un procédé qui permet de sélectionner, à partir d'un ensemble d'acides nucléiques de séquences distinctes de départ, un ou plusieurs acides nucléiques qui se fixent spécifiquement sur une molécule cible d'intérêt, par exemple un polypeptide cible d'intérêt. Pour mettre en oeuvre la méthode SELEX afin de sélectionner un acide nucléique ligand de la E-cadhérine, l'homme du métier se référera avantageusement au contenu des brevets américains n° US 5,475,096 et n° US 5,270,163.

A titre illustratif, un polypeptide ligand de la E-cadhérine peut être aisément sélectionné par l'homme du métier, par exemple en mettant en oeuvre des techniques de double-hybride bien connues, notamment décrites dans le brevet américain n° US 5,667,973.

Egalement, un polypeptide ligand de la E-cadhérine peut être aisément sélectionné à l'aide de techniques utilisant un biocapteur optique, telles que celle décrite par Edwards et Leatherbarrow (1997, Analytical Biochemistry, 246 : 1-6) ou encore par Szabo et al. (1995, Curr. Opinion Struct. Biol., 5(5) : 699-705). Cette technique permet la détection des interactions entre deux molécules en temps réel, sans nécessiter leur marquage préalable. Cette technique est basée sur le phénomène de résonance de surface du plasma (ou SPR pour « Surface Plasmon Resonance »). Brièvement, une première molécule partenaire, par exemple la E-cadhérine, est immobilisée sur une surface telle qu'une matrice de carboxyméthyl dextran. Puis, une seconde molécule partenaire, par exemple un polypeptide test, est incubé avec la première molécule partenaire, puis on détecte, par SPR, la fixation ou l'absence de fixation, et le cas échéant le niveau de fixation, entre les deux molécules, par exemple entre la E-cadhérine et le polypeptide test.

Un exemple de polypeptide ligand de la E-cadhérine est la beta-caténine, par exemple la beta-caténine de séquence en acides aminés SEQ ID N°2.

Un autre exemple de polypeptide ligand de la E-cadhérine consiste en un anticorps dirigé spécifiquement contre la E-cadhérine, et plus spécifiquement un anticorps dirigé contre le domaine extra-cellulaire de la E-cadhérine.

Les anticorps selon l'invention sont des anticorps polyclonaux ou monoclonaux.

De préférence, les anticorps polyclonaux sont choisis parmi :
(i) une fraction totale d'anticorps purifiés à partir du sérum d'un mammifère immunisé à l'encontre de la E-cadhérine ;
(ii) une fraction purifiée d'anticorps polyclonaux monospécifiques dirigés contre la E-cadhérine ;
(iii) les fragments Fab ou F(ab)'2 préparés à partir des anticorps polyclonaux (i) et (ii) ci-dessus.

L'obtention d'une fraction totale d'anticorps (i) purifiés à partir du sérum d'un mammifère immunisé à l'encontre de la E-cadhérine humaine comprend de préférence les étapes suivantes :
a) l'injection à un mammifère d'une composition comprenant la E-cadhérine ou un polypeptide comprenant le domaine extra-cellulaire de la E-cadhérine, en combinaison avec au moins un adjuvant de l'immunité ;
b) récupération de l'immunsérum dudit mammifère, contenant les anticorps dirigés contre la E-cadhérine,
c) La purification d'une fraction d'anticorps à partir de l'immunsérum.

Chacune de ces étapes est décrite plus en détail ci-dessous. La récupération de l'immunsérum est réalisée d'une manière connue de l'homme du métier, par exemple par séparation du sérum par centrifugation d'un échantillon de sang total. La purification d'une fraction d'anticorps à partir de l'immunsérum d'un patient immunisé par exemple par chromatographie d'affinité, précipitation au sulfate d'ammonium, chromatographie échangeuse d'ions, filtration sur gel, chromatographie sur colonne de Protéine A / G, chromatographie d'affinité, ou chromatographie d'immunoaffinité.

Une fraction purifiée d'anticorps polyclonaux monospécifiques (ii) dirigés contre la E-cadhérine peut être obtenue en réalisant une chromatographie d'affinité à partir d'une fraction totale d'anticorps (i) purifiés à partir du immunsérum d'un mammifère immunisé à l'encontre la E-cadhérine humaine, en fixant la E-cadhérine ou un motif antigénique de la E-cadhérine sur la colonne.

La purification des fragments F(ab')₂ à partir des anticorps polyclonaux décrits ci-dessus ou à partir de l'immunsérum ou du plasma sanguin peut être conduite selon la méthode décrite dans la demande de brevet US2002/0164327, comprenant une étape de digestion du plasma sanguin ou du sérum par la pepsine et des étapes de séparation et de purification jusqu'à obtention de fragments F(ab')₂, dépourvus d'albumine, d'anticorps complets, et substantiellement dépourvus de substances pyrogènes.

L'isolement de fractions F(ab) et F(ab')₂ permet d'obtenir des avantages spécifiques, comme le fait de se fixer à la E-cadhérine sans pour autant interagir avec d'autres molécules effectrices du système immunitaire.

Les fragments F(ab), peuvent être obtenus par une méthode similaire, consistant à digérer l'immunsérum, le plasma ou les fractions purifiées d'anticorps polyclonaux (i), (ii) et (iii) provenant d'un mammifère immunisé contre la E-cadhérine, par la papaïne.

Alternativement, les anticorps sont monoclonaux et choisis parmi :
(i) des anticorps produits par des cellules issues de la fusion cellulaire entre (a) des cellules B d'un mammifère immunisé à l'encontre de la E-cadhérine humaine et (b) des cellules d'une lignée cellulaire productrice d'anticorps, telle que des cellules d'un myélome ;
(ii) des anticorps produits par des cellules transfectées ou transformée avec un ADN codant une immunoglobuline, ledit ADN étant préalablement isolé à partir de l'ADN d'une cellule B d'un mammifère immunisé à l'encontre de la E-cadhérine humaine ;
(iii) les fragments Fab ou F(ab)'2 préparés à partir des anticorps polyclonaux (i) et (ii) ci-dessus ;
(iv) des fragments ScFv .

Selon l'invention, on peut utiliser l'un quelconque des nombreux anticorps monoclonaux ou polyclonaux qui reconnaissent la E-cadhérine humaine qui sont accessibles dans le commerce. Notamment, on peut utiliser les anticorps anti-E-cadhérine commercialisés par la société Zymed, notamment les anticorps contenus dans le kit ELISA référencé n° 99-1700. On peut aussi utiliser les anticorps monoclonaux anti-E-cadhérine commercialisés par la société Axxora sous les références n° ECCD-2, HECD-1 ou n° SHE78-7, ou encore ceux commercialisés par la société Biocare Medical sous les références n° CM170A, CM170B, CM170C et PM170AA, ou encore ceux commercialisés par la société Serotec sous le n° MCA1482, ou encore ceux commercialisés par la société Acris Antibodies GmbH sous le n°SM1531P, ou encore ceux commercialisés par la société sous le n° MAB3199, ou encore ceux commercialisés par la société DbioSys sous le n° Mob 193. On peut aussi utiliser les anticorps polyclonaux anti-E-cadherine commercialisés par la société Novus Biologicals sous la référence n° ab15148, ou encore ceux commercialisés par la société AbCam sous le n° ab14015.

On utilise de préférence des anticorps polyclonaux ou monoclonaux qui se fixent sélectivement sur la partie extra-cellulaire de la E-cadhérine.

Avantageusement, on utilise l'anticorps monoclonal anti-E-cadhérine commercialisé par la société Zymed sous la référence n° 13-1700. Cet anticorps monoclonal reconnaît le domaine EC1 de la partie extra-membranaire de la E-cadhérine, qui est localisé de l'acide aminé en position 157 jusqu'à l'acide aminé en position 262 de la séquence d'acides aminés SEQ ID N°1.

Dans certains modes de réalisation de l'étape c) du procédé, on utilise un composé ligand secondaire qui a la capacité de se fixer sur le composé ligand de la E-cadhérine, ledit composé ligand secondaire étant marqué par une molécule détectable.

Dans certains modes de réalisation de l'étape c) du procédé, la détection de la E-cadhérine est effectuée en mettant en contact les cultures de cellules avec un composé secondaire marqué par une molécule détectable, ledit composé secondaire marqué ayant la capacité de se fixer sur le composé ligand de la E-cadhérine. Selon ce mode de réalisation, la détection des complexes formés entre la E-cadhérine présente à la surface des membranes cellulaires et le composé ligand de la E-cadhérine est réalisée par détection du signal émis par le composé secondaire marqué qui se fixe sur le composé ligand impliqué dans les complexes (E-cadhérine / composé ligand de la E-cadhérine). A titre illustratif d'un tel mode de réalisation, le composé secondaire marqué peut consister en un anticorps marqué dirigé spécifiquement contre le composé ligand de la E-cadhérine.

Dans un mode de réalisation particulier du procédé, le composé ligand de la E-cadhérine est marqué par une molécule détectable.

Selon la nature de la molécule détectable, la présence de la E-cadhérine sur les membranes cellulaires peut être suivie par des techniques connues en soi, notamment des techniques de mesure de fluorescence à l'aide, soit d'un cytomètre de flux, soit d'un lecteur de microplaques, soit d'un fluorimètre, soit grâce à un microscope à fluorescence, ou encore par des techniques colorimétriques, enzymatiques, de mesure de radioactivité ou des techniques immunologiques. A titre illustratif, la molécule détectable peut être choisie parmi un antigène, une protéine fluorescente, une protéine radioactive, une protéine réceptrice comme la biotine ou une protéine ayant une activité enzymatique.

Lorsque la molécule détectable consiste en un antigène, elle peut être tout type d'antigène, dès lors que des anticorps spécifiques de cet antigène sont déjà accessibles ou, alternativement, peuvent être préparés, selon toute technique d'obtention d'anticorps, notamment d'anticorps polyclonaux ou monoclonaux, bien connues de l'homme du métier. Préférentiellement, dans ce cas, la molécule détectable consiste en un antigène de faible taille. Ainsi, préférentiellement, on utilise, comme antigène, un peptide ayant une chaîne de 7 à 100 acides aminés de longueur, mieux de 7 à 50 acides aminés de longueur, et encore mieux de 7 à 30 acides aminés de longueur, par exemple 10 acides aminés de longueur. A titre illustratif, on peut utiliser l'antigène HA de séquence [NH₂-YPYDVPDYA-COOH] SEQ ID N° 3, ou encore un antigène FLAG de séquence [NH₂-DYKDDDDK-COOH] SEQ ID N° 4 (monomère FLAG) ou de séquence [NH₂-MDYKDHDGDYKDHDIDYKDDDDK-COOH] SEQ ID N° 5 (trimère FLAG) ou encore un antigène MYC de séquence [NH₂-MEQKLISEEDL-COOH] SEQ ID N°6. Dans ce cas, on utilise, pour quantifier la molécule détectable à l'étape (c) du procédé, un anticorps qui reconnaît spécifiquement l'antigène compris dans le composé ligand, cet anticorps étant marqué directement ou indirectement. La quantification est alors réalisée par mesure du signal détectable produit par les complexes formés, dans les préparations cellulaires, entre l'anticorps marqué et le composé ligand de la E-cadhérine comprenant le marqueur antigène.

Lorsque la molécule détectable consiste en une protéine à fluorescence intrinsèque, elle est notamment choisie parmi la protéine GFP ou l'un de ses dérivés, la protéine YFP ou l'un de ses dérivés, et la protéine dsRED. Parmi les protéines dérivées de la protéine GFP, on peut utiliser notamment l'une quelconque des protéines connues sous les noms GFPMut3, Venus, Sapphire etc. On peut notamment utilisée l'une des protéines à fluorescence intrinsèque décrites dans les brevets américains n° US 5,625,048, US 5,777,079, US 5,804,387, US 5,968,738, US 5,994,077, US 6,054,321, US 6,066,476, US 6,077,707, US 6,090,919, US 6,124,128, US 6,172,188, ou encore les brevets européens n° EP 851 874 et EP 804 457.

Lorsque la protéine détectable consiste en une protéine à fluorescence intrinsèque, on quantifie la protéine détectable à l'étape (b) du procédé par mesure du signal de fluorescence qui est émis par la protéine de fusion lκBα-protéine fluorescente à l'aide de tout dispositif adapté. Ainsi, à l'étape (b), lorsque la première protéine détectable est une protéine fluorescente, on quantifie ladite protéine détectable par une mesure du signal de fluorescence émis par ladite protéine.

Lorsque la protéine détectable consiste en une protéine à activité enzymatique, ladite protéine détectable est choisie notamment parmi la luciférase et la β-lactamase. Dans ce cas, on quantifie la molécule détectable à l'étape (c) du procédé par mesure de la quantité du ou des composés produits par la conversion du substrat par l'enzyme . Lorsque le produit de l'activité enzymatique est coloré, la mesure peut être réalisée par colorimétrie. Lorsque le produit de l'activité enzymatique est fluorescent, on mesure l'intensité du signal de fluorescence qui est émis par ledit produit, à l'aide de tout dispositif de mesure de la fluorescence adapté. Ainsi, à l'étape (c), lorsque la molécule détectable est une protéine ayant une activité enzymatique, on quantifie ladite protéine détectable par une mesure de la quantité de substrat transformé par ladite protéine, en général par mesure de la densité optique à la longueur d'onde d'émission du substrat.

Dans certains modes de réalisation du procédé de l'invention, le composé ligand consiste en un anticorps anti E-cadhérine qui est couplé à la biotine ou un anticorps secondaire dirigé contre le mammifère chez lequel a été produit l'anticorps anti E-cadhérine et couplé à la biotine. Dans ces modes de réalisation particuliers, on peut utiliser un composé secondaire marqué comprenant de la streptavidine. Un exemple illustratif d'un tel composé secondaire marqué est une protéine de fusion entre la streptavidine et une molécule détectable choisie parmi les molécules détectables définies ci-dessus, y compris une molécule détectable consistant en une enzyme. Par exemple, on peut utiliser un composé secondaire marqué consistant en une molécule de streptavidine couplée ou fusionnée avec la peroxydase de raifort ou le système de complexe soluble peroxydase anti-peroxydase (PAP) (commercialisé par la société SIGMA, P3039). Selon ce dernier système, le composé secondaire est composite. Ce composé secondaire composite comprend (i) un anticorps dirigé contre un anticorps anti E-cadhérine, et plus spécifiquement contre la partie Fc dudit anticorps anti E-cadhérine et (ii) un anticorps dirigé contre une peroxydase, telle que la peroxydase de raifort ou la peroxydase de soja, ledit anticorps étant complexé avec ladite peroxydase, au niveau du domaine de reconnaissance de l'antigène (CDR). Dans ce système, l'anticorps dirigé contre l'anticorps anti E-cadhérine peut être par exemple un anticorps anti-anticorps de souris, lorsque l'anticorps anti E-cadhérine consiste en un anticorps de souris. Selon ce système, deux molécules de peroxydase sont fixées sur l'anticorps anti-peroxydase, ce qui constitue un avantage technique et confère au procédé un seuil de sensibilité accru, par rapport à de nombreux autres systèmes de marquage et de détection.

Ainsi, selon ce système, on peut réaliser la détection de la E-cadhérine exposée à la surface membranaire des cellules à l'aide (i) d'un anticorps anti E-cadhérine, sur lequel on fixe (ii) un anticorps dirigé contre l'anticorps anti E-cadhérine, sur lequel on fixe (iii) un anticorps anti-peroxydase complexé à la peroxydase au niveau de son site de reconnaissance de l'antigène (CDR).

On peut révéler la présence de complexes entre la E-cadhérine et un ligand de la E-cadhérine marqué à la peroxydase, fixés à la surface des membranes cellulaires, par la détection optique du produit résultant de la conversion d'un substrat de la peroxydase de raifort, comme l'ortho-phénylène diamine (OPD), après mise en contact du composé secondaire marqué avec les complexes E-cadhérine / ligand.

La présente invention a encore pour objet une trousse ou kit pour le criblage de substances anti-cancéreuses anti-métastatiques capables d'inverser le phénotype métastatique vers un phénotype non-métastatique comprenant :
a) des cellules métastatiques n'exprimant pas la E-cadhérine sur leur membrane cellulaire ;
b) un composé ligand de la E-cadhérine.

Les cellules tumorales incluses dans le kit de l'invention peuvent être choisies parmi les cellules tumorales décrites dans la présente description.

Avantageusement, le kit comprend des cellules tumorales ayant la capacité de former des métastases qui sont choisies parmi les lignées cellulaires suivantes : SW620 (ATCC n**°** CCL-227), SKCo-1 (ATCC n° HTB-39), et CoLo205 (ATCCn° CCL-222).

Dans un kit selon l'invention, le composé ligand de la E-cadhérine peut être l'un quelconque des composés ligands de la É-cadhérine décrits dans la présente description.

Avantageusement, on utilise un composé ligand de la E-cadhérine qui se fixe sur la partie extra-membranaire de la E-cadhérine.

Selon un mode de réalisation particulier du kit ci-dessus, le composé ligand de la E-cadhérine consiste en un anticorps polyclonal ou monoclonal anti E-cadhérine.

Sont englobés dans l'ensemble des anticorps anti E-cadhérine susceptibles d'être inclus dans un kit selon l'invention, les anticorps couplés à la biotine.

Dans certains modes de réalisation d'un kit de criblage de l'invention, ledit kit comprend de plus un composé ligand secondaire d'un type décrit précédemment dans la présente description. Ledit composé ligand secondaire peut consister en un composé ligand secondaire qui se fixe sur le composé ligand de la E-cadhérine, ledit composé ligand secondaire étant marqué par une molécule détectable.

Avantageusement, lorsque le composé ligand de la E-cadhérine consiste en un anticorps anti E-cadhérine couplé à la biotine, le composé ligand secondaire est choisi parmi les composés issus du couplage entre la streptavidine et une molécule détectable, par exemple entre la streptavidine et une enzyme. A titre illustratif, on peut utiliser, comme composé ligand secondaire, un composé résultant du couplage entre la streptavidine et la peroxydase de raifort.

Selon un autre mode de réalisation du kit selon l'invention, le composé ligand secondaire consiste en un anticorps dirigé contre l'anticorps anti E-cadhérine, par exemple anticorps dirigé contre l'anticorps anti E-cadhérine qui est marqué par une molécule détectable.

Selon un mode de réalisation particulier d'un anticorps anti E-cadhérine marqué par une molécule détectable, on utilise un anticorps anti E-cadhérine, sur lequel est fixé (ii) un anticorps anti-peroxydase qui est reconnu par l'anticorps dirigé contre l'anticorps anti-E-cadhérine, ledit anticorps anti-peroxydase étant lié à deux molécules de peroxydase au niveau de son site de liaison à l'antigène (CDR).

La présente invention est aussi relative à l'utilisation d'un composé ligand de la partie extra-membranaire de la E-cadhérine, pour le criblage *in vitro* de substances anti-cancéreuses anti-métastatiques capables d'inverser le phénotype métastatique vers un phénotype non-métastatique. De préférence, le composé ligand est utilisé conjointement avec une culture de cellules tumorales n'exprimant pas la E-cadhérine sur leur membrane cellulaire.

La présente invention est également relative à l'utilisation d'un inhibiteur de la kinase ROCK (« Rho-associated coiled-coil forming protein serine/threonine kinase») pour la fabrication d'une composition pharmaceutique destinée à la prévention ou au traitement d'un cancer, notamment d'un cancer choisi parmi ceux définis dans la présente description. Plus particulièrement, un inhibiteur de la protéine kinase ROCK est utilisé pour la fabrication d'un médicament anti-métastatique.

Un inhibiteur de la kinase ROCK peut être choisi dans le groupe constitué deY-27632 commercialisé par la société VWR INTERNATIONAL (REF :688 000-5).

La présente invention est en outre illustrée par les figures et les exemples suivants.

### FIGURES

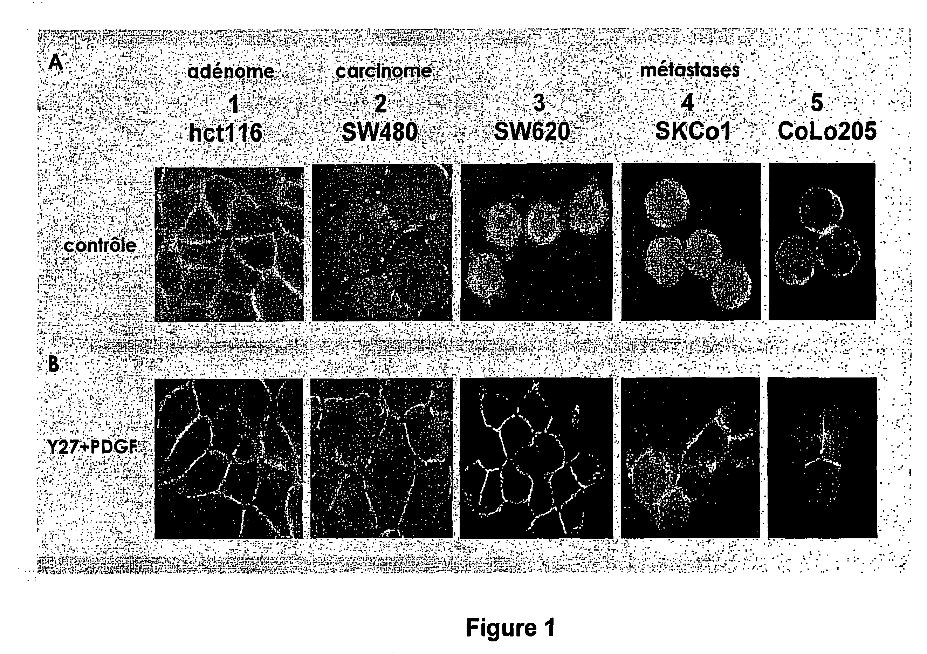

La **Figure 1** illustre l'effet de l'inhibiteur Y-27632, qui est un inhibiteur de la kinase ROCK, sur la relocalisation correcte de la E-cadhérine au niveau des jonctions inter-cellulaires dans plusieurs lignées de cellules cancéreuses. Figures A1 à A5 : cellules témoins sans inhibiteur. Figures B1 à B5 : cellules incubées avec l'inhibiteur Y-27632. Colonnes 1 et 2 : cellules cancéreuses non-métastatiques hct116 (1) et SW480 (2). Colonnes 3 à 5: cellules cancéreuses métastatiques SW620(3), SKCo-1(4) et CoLo205(5).

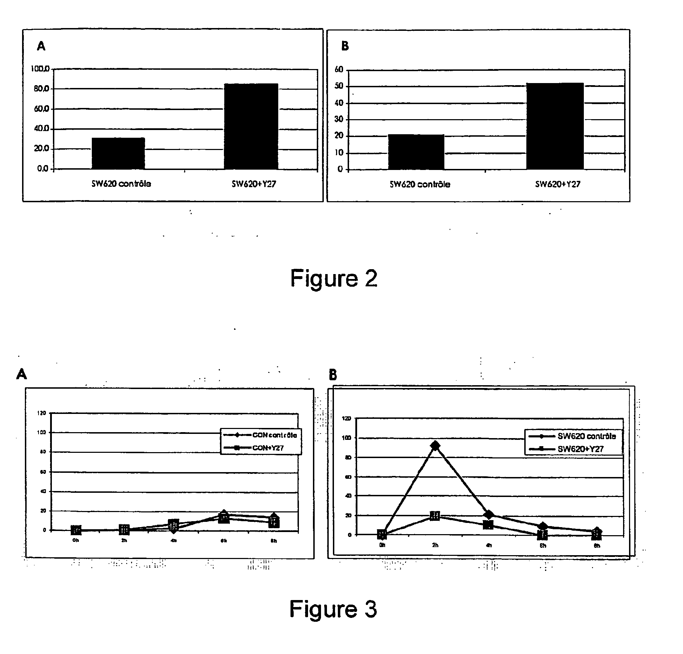

La **Figure 2** illustre l'effet du traitement des cellules métastatiques de la lignée SW620 par l'inhibiteur Y-27632, sur l'étalement des cellules (Figure 2A) et sur la formation de jonctions adhérentes de type E-cadhérine (Figure 2B). Sur les Figures 2 A et 2 B : à gauche : cellules témoins sans inhibiteur ; à droite : cellules incubées avec Y-27632. En abscisse : pourcentage de cellules étalées (Figure 2A) ou pourcentage de cellules formant des jonctions intercellulaire de type E-cadhérine (Figure 2B).

La **Figure 3** illustre une comparaison de l'effet du traitement par l'inhibiteur Y-27632 des cellules de colon normales de la lignée CoN (Figure 3A) et des cellules métastatiques de la lignée SW620 (Figure 3B) sur le pouvoir invasif de ces cellules. En abscisse : temps de migration en présence ou non de Y-27632. En ordonnées : moyenne du nombre de cellules par champ ayant traversé la matrice cellulaire reconstituée (microscope, OBJ x 20).

### EXEMPLES

### EXEMPLE 1: Identification d'une corrélation stricte entre la formation de jonctions intercellulaires de type E-cadhérine et la réversion de cellules cancéreuses vers le phénotype non-métastatique.

### A. MATERIEL ET METHODES

### 1. Protocole d'immunofluorescence marquage de la e-Cadherine

1- Les cellules sont ensemencées dans des plaques 6 puits, chaque puits contenant 3 lamelles de verre de 10mm de diamètre.
2- Placer les cellules en 0% Sérum pendant 18 à 24 heures puis traiter ou non avec Y27 10µM + PDGF 5ng/ml pendant 36 à 48h.
3- Fixer les cellules 10 minutes à la formalin (Formadéhyde 3,7% dans PBS)
4- Saturation, 10 minutes PBS-BSA
5- Incubation de l'anticorps primaire, dilué au 1:500 dans PBS-BSA, 2 heures à 37°C (Anti E-cadhérine de souris, ZYMED, ref : 13-1700)
6- 2 rinçages rapides au PBS - 0.1% Tween
7- Incubation de l'anticorps secondaire (Alexa Fluod® 488 F(ab')₂ fragment of goat anti mouse IgG, Molecular Probes ref : A-11017), dilué au 1:2000 dans PBS-BSA, 30 minutes à 37°C.
8- 2 rinçages rapides au PBS - 0.1 % Tween
9- Montage dans du milieu Mowiol entre lame et lamelle.

### 2. Test d'invasion

### 1- à J-2, décongeler le matrigel à 4°C toute la nuit...

### 2- à J-1, couler le matrigel dans les nacelles :

Placer l'ensemble du matériel nécessaire dans la glace (matrigel, milieu de culture, cônes, eppendorfs, etc...).
Placer les inserts fluoroblocks (FALCON, ref : 351152) dans les plaques 24 puits (FALCON, ref : 353504).
Diluer le matrigel à 2mg/ml dans du milieu de culture sans sérum froid (si les cellules doivent subir un traitement X, inclure le produit dans le matrigel, ex : Y 27632)
Répartir 100 µl de matrigel 2mg/ml par insert en évitant de faire des bulles...
Laisser O/N à 37°C dans une atmosphère humide (bref dans l'incubateur, quoi !!!)

### 3- Le jour J, ensemencer les cellules sur le matrigel :

Dans un premier temps, mettre 700µl de milieu 10% sérum dans une plaque 24
puits et transférer les inserts dans cette plaque.

Trypsiner les cellules (préalablement traitées ou non...) et les reprendre dans du milieu 2% sérum.

Ensemencer 50.000 cellules par insert dans 200µl de milieu 2% sérum sur le matrigel (mettre éventuellement du Y27632 dans les cellules ensemencées).

### 4- Le temps de migration est variable en fonction du type cellulaire mais généralement laisser migrer 8 heures en fixant un point toutes les 2 heures semble convenable.

Pour fixer, transférer les inserts dans une plaque 24 puits contenant 1 ml de formalin (Formaldéhyde 3,7% dans PBS), aspirer le milieu de culture à l'intérieur des nacelles et ajouter également de la formalin (très important, évite aux cellules de continuer à migrer dans le matrigel !!!). Incuber 10 minutes.
Faire 3 rinçages rapides au PBS

Ensuite, réaliser le marquage au Iodure de propidium en incubant les inserts dans 1 ml de Iodure de propidium (SIGMA, ref : P-4864) 1:500 dans PBS, O/N à 4°C, à l'abris de la lumière.

### 3. Protocole de marquage e-cadherine en plaques 96 puits

1- Ensemencer environ 10.000 cellules par puits pour des lignées colorectales (dans des plaques 96 puits Falcon, ref : 353072).
2- Faire les traitements nécessaires (contrôle, Y27, PDGF...pendant 48h) et fixer 10 minutes à la formalin (Formadéhyde 3,7% dans PBS)
3- Saturation, 10 minutes PBS-BSA
4- Incubation de l'anticorps primaire, dilué au 1:500 dans PBS-BSA, 2 heures à 37°C (Anti E-cadhérine de souris, ZYMED, ref : 13-1700)
5- 2 rinçages rapides au PBS - 0.1 % Tween
6- Incubation Ac anti-mouse biotynilé, dilution 1:1000 dans PBS-BSA, 30 minutes à 37°C, (Anti-Mouse IgG, Heavy and light chain specific biotin conjugate, CALBIOCHEM, ref : 401213)
7- 2 rinçages rapides au PBS - 0.1% Tween
8- Incubation Streptavidine-HRP, dilution entre 1:1000 dans PBS-BSA, 30 minutes à 37°C, (ECL strptavidine-Horseradish Peroxidase conjugate, AMERSHAM BIOSCIENCES, ref : RPN1231)
9- 2 rinçages rapides au PBS - 0.1% Tween
10- Révélation :
   Dissoudre une capsule de « Phosphate-citrate buffer containing sodium perborate » (SIGMA, ref: P4922) dans 100 ml d'eau désionisée. Ce tampon doit être utilisé dans les 30 minutes qui suivent sa reconstitution.
   Dissoudre des tablettes d'o-Phenylenediamine (OPD) dihydrochloride (SIGMA, ref : P6787) dans ce tampon pour obtenir une concentration finale de 0.4 mg/ml (1 pastille d'OPD à 10 mg dans 40 ml de Tampon perborate).
   Mettre 100 µl d'OPD/Perborate par puits et incuber à température ambiante pendant 5 minutes.
   Stopper la réaction avec 50 µl de HCI 3N.
   Lire la DO à 490 nm.

### B. RESULTATS

On a montré que le phénotype des cellules métastasiques SW620, c'est-à-dire une morphologie sphérique associée à l'absence de jonctions de type E-cadhrérine et à un pouvoir invasif élevé, pouvait être réversé par un inhibiteur de la kinase ROCK, le Y-27632. Cette kinase est un puissant activateur de la migration et a déjà été impliqué dans des phénomènes invasifs. On a montré que le traitement des cellules de la lignée métastasique SW620 avec le Y-27632 favorise l'étalement de ces cellules (figure 2A) et la formation de jonction E-cadhérine dépendantes (figure 1 B et figure 2B). On a montré que le traitement de ces cellules au Y-27632 permet également de réduire drastiquement leur capacité d'invasion. En effet, après traitement avec le Y-27632, les cellules métastasiques SW620 retrouvent un niveau d'invasion très faible, comparable à celui des cellules de colon normales CoN (figure 3B).

Comme illustré sur la figure 1, le traitement des cellules colorectales avec l'inhibiteur de la kinase Rock (Y-27632) induit une relocalisation correcte de la E-cadhérine au niveau des jonctions dans les lignées de cellules métastasiques (lignées SW620, SKCo-1 et CoLo205). La figure 1A illustre la localisation de la E-cadhérine dans différentes lignées de cellules colorectales contrôle. La figure 1B illustre la localisation de la E-cadhérine dans différentes lignées de cellules colorestales traitées avec l'inhibiteur de la kinase Rock (Y-27632, 10 µM pendant 48 heures).

Comme illustré sur la figure 2, le traitement des cellules de la lignée métastasique SW620 avec l'inhibiteur de la kinase Rock (Y-27632) induit l'étalement des cellules et la formation de jonction de type E-cadhérine. Sur la figure 2A, les cellules SW620 témoins présentent à 30% une morphologie étalée et à 70% une morphologie sphérique. Après traitement au Y-27632, 90% des cellules sont étalées et seulement 10% conservent une morphologie sphérique. Sur la figure 2B, 22% des cellules SW620 témoins sont capables de former des jonctions. Après traitement au Y-27632, 52%, les cellules sont capables de former des jonctions de type E-cadhérine.

En conclusion, les tests de formation de jonctions E-cadhérine dépendantes (figure 2) menés parallèlement aux tests d'invasion (figure 3) ont permis de mettre en évidence, dans le modèle de cellules de métastases de colon SW620, qu'il existe une corrélation stricte entre capacité de reformation de jonctions de type E-cadhérine et diminution de la capacité d'invasion.

### EXEMPLE 2 : Illustration d'un mode de réalisation du procédé de criblage selon l'invention.

1 - Ensemencer environ 10.000 cellules par puits pour des lignées colorectales dans des plaques 96 puits Falcon, (ref: 3530072).
2 - Faire les traitements nécessaires (contrôle, Y27, PDGF ... pendant 48 heures) et fixer 10 minutes à la formaline (Formaldéhyde de 3,7% dans PBS).
3 - Saturation, 10 minutes PBS-BSA
4 - Incubation de l'anticorps primaire, dilué au 1:500 dans PBS-BSA, 2 heures à 37°C (Anti E-cadhérine de souris, ZYMED, ref:13-1700).
5-2 rinçages rapides au PBS - 0,1% Tween.
6 - Incubation des anticorps anti-souris biotynilé, dilution 1:1000 dans PBS-BSA, 30 minutes à 37°C. (Anti-Mouse IgG, Heavy and light chain specific biotin conjugate, CALBIOCHEM, ref: 401213).
7-2 rinçages rapides au PBS - 0.1 % Tween.
8 - Incubation Streptavidine-HRP, dilution entre 1:1000 dans PBS-BSA, 30 minutes à 37°C, (ECL streptavidine-Horseradish Peroxidase conjugate, AMERSHAM BIOSCIENCES ref: RPN1231).
9-2 rinçages rapides au PBS - 0, 1 % Tween.
10 - Révélation:
   a) Dissoudre une capsule de "Phosphate-citrate buffer containing sodium perborate" (SIGMA, ref: P4922) dans 100 ml d'eau désionisée. Ce tampon doit être utilisé dans les 30 minutes qui suivent sa reconstitution.
   b) Dissoudre des tablettes d'o-Phenylenediamine (OPD) dihydrochloride (SIGMA, ref: P6787) dans ce tampon pour obtenir une concentration finale de 0,5 mg/ml (1 pastille d'OPD à 10 mg dans 40 ml de Tampon perborate).
   c) Mettre 100 µm d'OPD/perborate par puits et incuber à température ambiante pendant 5 minutes.
   d) Stopper la réaction avec 50 µm de HCI 3N.
   e) Lire la DO à 490 nm.

## Revendications

1. Procédé pour le criblage de substances anti-cancéreuses anti-métastatiques capables d'inverser le phénotype métastatique vers un phénotype non métastatique comprenant les étapes suivantes :
a) cultiver des cellules métastatiques n'exprimant pas la E-cadhérine sur leur membrane cellulaire ;
b) mettre en contact les cellules cultivées à l'étape a) avec une substance ou une combinaison de substances candidate(s) ;
c) détecter, dans la culture de cellules obtenue à la fin de l'étape b), la présence de E-cadhérine à la surface cellulaire ;
d) sélectionner positivement la substance ou la combinaison de substances candidate(s) lorsque la E-cadhérine a été détectée à l'étape c).

2. Procédé selon la revendication 1, **caractérisé en ce qu'**à l'étape c), la détection est réalisée en mettant en contact les cellules obtenues à la fin de l'étape b) avec un composé ligand de la E-cadhérine.

3. Procédé selon la revendication 2, **caractérisé en ce que** le composé ligand de la E-cadhérine se fixe sur la partie extra-membranaire de la E-cadhérine.

4. procédé selon l'une des revendications 2 ou 3, **caractérisé en ce que** le composé ligand est marqué par une molécule détectable.

5. Procédé selon l'une des revendications 2 à 4, **caractérisé en ce qu'**à l'étape c), on utilise un composé ligand secondaire qui se fixe sur le composé ligand de la E-cadhérine, ledit second composé ligand étant marqué par une molécule détectable.

6. Procédé selon l'une des revendications 2 à 5, **caractérisé en ce que** le composé ligand consiste en un anticorps anti-E-Cadhérine.

7. Procédé selon la revendication 6, **caractérisé en ce qu'**à l'étape c), on utilise un composé ligand secondaire qui se fixe sur l'anticorps anti-E-cadhérine, ledit second composé ligand étant marqué par une molécule détectable.

8. Procédé selon la revendication 7, **caractérisé en ce que** le composé ligand secondaire comprend une molécule de streptavidine couplée ou fusionnée à une enzyme.

9. Procédé selon la revendication 7, **caractérisé en ce que** le second composé ligand est un anticorps dirigé contre l'anticorps anti-E-cadhérine.

10. Procédé selon l'une des revendications 1 à 9, **caractérisé en ce que** les cellules métastatiques sont choisies parmi les lignées cellulaires suivantes : SW620 (ATCC n° CCL-227), SKCo-1 (ATCC n° HTB-39) et CoLo205 (ATCC n° CCL-222).

11. Trousse ou kit pour le criblage de substances anti-cancéreuses anti-métastatiques capables d'inverser le phénotype métastatique vers un phénotype non métastatique comprenant :
a) des cellules métastatiques n'exprimant pas la E-cadhérine sur leur membrane cellulaire ;
b) un composé ligand de la E-cadhérine.

12. Trousse ou kit selon la revendication 11, **caractérisé en ce que** le composé ligand de la E-cadhérine se fixe sur la partie extra-membranaire de la E-cadhérine.

13. Trousse ou kit selon l'une des revendications 11 ou 12, **caractérisé en ce que** le composé ligand de la E-cadhérine consiste en un anticorps anti-E-cadhérine.

14. Trousse ou kit selon l'une des revendications 11 à 13, **caractérisé en ce qu'**il comprend de plus un composé ligand secondaire qui se fixe sur le composé ligand de la E-cadhérine, ledit second composé ligand étant marqué par une molécule détectable.

15. Trousse ou kit selon la revendication 14, **caractérisé en ce que** le second composé ligand est un anticorps dirigé contre l'anticorps anti-E-cadhérine.

16. Trousse ou kit selon la revendication 15, **caractérisé en ce que** le composé ligand secondaire consisté en un anticorps anti-peroxydase qui est reconnu par l'anticorps dirigé contre l'anticorps anti-E-cadhérine, ledit anticorps anti-peroxydase étant lié à deux molécules de peroxydase au niveau de son site de liaison à l'antigène (CDR).

17. Utilisation d'un composé ligand de la partie extra-membranaire de la E-cadhérine, pour le criblage *in vitro* de substances anti-cancéreuses anti-métastatiques capables d'inverser le phénotype métastatique vers un phénotype non métastatique.

18. Utilisation selon la revendication 17, **caractérisée en ce que** le composé ligand est utilisé conjointement avec une culture de cellules métastatiques n'exprimant pas la E-cadhérine sur leur membrane cellulaire

## Claims

1. A method for screening antimetastatic anticancer substances which are able to reverse the metastatic phenotype to a non-metastatic phenotype comprising the following steps:
a) growing metastatic cells which do not express E-cadherin on their cell membrane;
b) bringing the cultivated cells obtained at step a) into contact with a candidate substance or a combination of candidate substances;
c) detecting, in the culture of cells obtained at the end of step b), the presence of E-cadherin at the cell surface;
d) positively selecting the candidate substance, or the combination of candidate substances, when the E-cadherin has been detected at step c).

2. The method according to claim 1, **characterised in that** at step c), the detection is carried out by bringing the cells obtained at the end of step b) into contact with a ligand compound of E-cadherin.

3. The method according to claim 2, **characterised in that** the E-cadherin ligand compound binds to the extracellular portion of E-cadherin.

4. The method according to one of claims 2 or 3, **characterised in that** the ligand compound is labelled with a detectable molecule.

5. The method according to one of claims 2 or 4, **characterised in that** at step c), a secondary ligand compound that binds to the E-cadherin ligand compound is used, the said second ligand compound being labelled with a detectable molecule.

6. The method according to one of claims 2 or 5, **characterised in that** the ligand compound consists of an anti-E-cadherin antibody.

7. The method according to claim 6, **characterised in that** at step c), a secondary ligand compound that binds to the anti-E-cadherin antibody is used, the said second ligand compound being labelled with a detectable molecule.

8. The method according to claim 7, **characterised in that** the secondary ligand compound comprises a streptavidin molecule coupled to, or fused with, an enzyme.

9. The method according to claim 7, **characterised in that** the second ligand compound is an antibody directed against the anti-E-cadherin antibody.

10. The method according to one of claims 1 to 9, **characterised in that** the metastatic cells are selected from among the following cell lines: SW620 (ATCC n° CCL-227), SKCo-1 (ATCC n° HTB-39) and CoLo205 (ATCCn° CCL-222).

11. A set or kit for screening antimetastatic anticancer substances which are able to reverse the metastatic phenotype to a non-metastatic phenotype comprising:
a) metastatic cells which do not express E-cadherin on their cell membrane;
b) a ligand compound of E-cadherin.

12. A set or kit according to claim 11, **characterised in that** the E-cadherin ligand compound binds to the extracellular portion of E-cadherin.

13. A set or kit according to one of claims 11 or 12, **characterised in that** the E-cadherin ligand compound consists of an anti-E-cadherin antibody.

14. A set or kit according to one of claims 11 or 13, **characterised in that** it also contains a secondary ligand compound that binds to the E-cadherin ligand compound, the said second ligand compound being labelled with a detectable molecule.

15. A set or kit according to claim 14, **characterised in that** the second ligand compound is an antibody directed against the anti-E-cadherin antibody.

16. A set or kit according to claim 15, **characterised in that** the secondary ligand compound consists of an anti-peroxidase antibody that is recognised by the antibody directed against the anti-E-cadherin antibody, said anti-peroxidase antibody being bound to two peroxidase molecules at its antigen binding site (CDR).

17. The use of a ligand compound of the extracellular portion of E-cadherin for *in vitro* screening of antimetastatic anticancer substances which are able to reverse the metastatic phenotype to a non-metastatic phenotype.

18. The use, according to claim 17, **characterised in that** the ligand compound is used in conjunction with a culture of metastatic cells which do not express E-cadherin on their cell membrane.

## Patentansprüche

1. Verfahren zum Screening von antimetastatischen geschwulsthemmenden Substanzen, die den metastatischen Phänotyp zu einem nichtmetastatischen Phänotyp umkehren können, umfassend die folgenden Schritte:
a) Kultivieren von metastatischen Zellen, die kein E-Cadherin auf ihrer Zellmembran exprimieren;
b) In-Kontakt-Bringen der in Schritt a) kultivierten Zellen mit einer in Frage kommenden Substanz oder Kombination von Substanzen;
c) Nachweisen der Gegenwart von E-Cadherin auf der Zelloberfläche in der am Ende von Schritt b) erhaltenen Zellkultur;
d) positives Selektieren der in Frage kommenden Substanz oder Kombination von Substanzen, wenn E-Cadherin in Schritt c) nachgewiesen wurde.

2. Verfahren gemäß Anspruch 1, **dadurch gekennzeichnet, dass** der Nachweis in Schritt c) durch In-Kontakt-Bringen der am Ende von Schritt b) erhaltenen Zellen mit einer Ligandverbindung von E-Cadherin erfolgt.

3. Verfahren gemäß Anspruch 2, **dadurch gekennzeichnet, dass** die Ligandverbindung von E-Cadherin an den extramembranen Teil von E-Cadherin bindet.

4. Verfahren gemäß einem der Ansprüche 2 oder 3, **dadurch gekennzeichnet, dass** die Ligandverbindung mit einem nachweisbaren Molekül markiert ist.

5. Verfahren gemäß einem der Ansprüche 2 bis 4, **dadurch gekennzeichnet, dass** in Schritt c) eine sekundäre Ligandverbindung verwendet wird, die an die Ligandverbindung von E-Cadherin bindet, wobei die sekundäre Ligandverbindung mit einem nachweisbaren Molekül markiert ist.

6. Verfahren gemäß einem der Ansprüche 2 bis 5, **dadurch gekennzeichnet, dass** die Ligandverbindung aus einem Anti-E-Cadherin-Antikörper besteht.

7. Verfahren gemäß Anspruch 6, **dadurch gekennzeichnet, dass** in Schritt c) eine sekundäre Ligandverbindung verwendet wird, die an den Anti-E-Cadherin-Antikörper bindet, wobei die sekundäre Ligandverbindung mit einem nachweisbaren Molekül markiert ist.

8. Verfahren gemäß Anspruch 7, **dadurch gekennzeichnet, dass** die sekundäre Ligandverbindung ein Streptavidin-Molekül umfasst, das an ein Enzym gekoppelt oder damit fusioniert ist.

9. Verfahren gemäß Anspruch 7, **dadurch gekennzeichnet, dass** die sekundäre Ligandverbindung ein gegen den Anti-E-Cadherin-Antikörper gerichteter Antikörper ist.

10. Verfahren gemäß einem der Ansprüche 1 bis 9, **dadurch gekennzeichnet, dass** die metastatischen Zellen aus den folgenden Zelllinien ausgewählt sind: SW620 (ATCC-Nr. CCL-227), SKCo-1 (ATCC-Nr. HTB-39) und CoLo205 (ATCC-Nr. CCL-222).

11. Zusammenstellung oder Kit zum Screening von antimetastatischen geschwulsthemmenden Substanzen, die den metastatischen Phänotyp zu einem nichtmetastatischen Phänotyp umkehren können, umfassend:
a) metastatische Zellen, die kein E-Cadherin auf ihrer Zellmembran exprimieren;
b) eine Ligandverbindung von E-Cadherin.

12. Zusammenstellung oder Kit gemäß Anspruch 11, **dadurch gekennzeichnet, dass** die Ligandverbindung von E-Cadherin an den extramembranen Teil von E-Cadherin bindet.

13. Zusammenstellung oder Kit gemäß einem der Ansprüche 11 oder 12, **dadurch gekennzeichnet, dass** die Ligandverbindung von E-Cadherin aus einem Anti-E-Cadherin-Antikörper besteht.

14. Zusammenstellung oder Kit gemäß einem der Ansprüche 11 bis 13, **dadurch gekennzeichnet, dass** er außerdem eine sekundäre Ligandverbindung umfasst, die an die Ligandverbindung von E-Cadherin bindet, wobei die sekundäre Ligandverbindung mit einem nachweisbaren Molekül markiert ist.

15. Zusammenstellung oder Kit gemäß Anspruch 14, **dadurch gekennzeichnet, dass** die sekundäre Ligandverbindung ein gegen den Anti-E-Cadherin-Antikörper gerichteter Antikörper ist.

16. Zusammenstellung oder Kit gemäß Anspruch 15, **dadurch gekennzeichnet, dass** die sekundäre Ligandverbindung aus einem Anti-Peroxidase-Antikörper besteht, der von dem gegen den Anti-E-Cadherin-Antikörper gerichteten Antikörper erkannt wird, wobei der Anti-Peroxidase-Antikörper an seiner Antigen-Bindungsstelle (CDR) an zwei Moleküle Peroxidase gebunden ist.

17. Verwendung einer Ligandverbindung des extramembranen Teils von E-Cadherin zum in-vitro-Screening von antimetastatischen geschwulsthemmenden Substanzen, die den metastatischen Phänotyp zu einem nichtmetastatischen Phänotyp umkehren können.

18. Verwendung gemäß Anspruch 17, **dadurch gekennzeichnet, dass** die Ligandverbindung zusammen mit einer Kultur von metastatischen Zellen, die kein E-Cadherin auf ihrer Zellmembran exprimieren, verwendet wird.
